(19) Europäisches Patentamt / European Patent Office / Office européen des brevets

(11) **EP 1 506 769 B1**

(12) **FASCICULE DE BREVET EUROPEEN**

(45) Date de publication et mention
de la délivrance du brevet:
**24.01.2007 Bulletin 2007/04**

(51) Int Cl.:
*A61K 8/11* *(2006.01)*          *A61K 8/81* *(2006.01)*
*A61Q 5/06* *(2006.01)*

(21) Numéro de dépôt: **04292023.1**

(22) Date de dépôt: **10.08.2004**

(54) **Utilisation de micro-objets adhésifs non-sphériques pour le coiffage et/ou le maquillage des cheveux**

Verwendung von nicht-sphärischen haftenden Mikrokörpern zum Frisieren und/oder Schminken der Haare

Use of non-spherical adhesive micro-objects for styling and/or coiffing hair

(84) Etats contractants désignés:
**AT BE BG CH CY CZ DE DK EE ES FI FR GB GR HU IE IT LI LU MC NL PL PT RO SE SI SK TR**

(30) Priorité: **13.08.2003 FR 0309917**

(43) Date de publication de la demande:
**16.02.2005 Bulletin 2005/07**

(73) Titulaire: **L'ORÉAL**
**75008 Paris (FR)**

(72) Inventeurs:
• **Rollat, Isabelle**
**75017 Paris (FR)**
• **Samain, Henri**
**91570 Bievres (FR)**

(74) Mandataire: **Casalonga, Axel**
**Bureau Casalonga & Josse**
**Bayerstrasse 71/73**
**80335 München (DE)**

(56) Documents cités:
**EP-A- 1 072 650          US-A- 5 441 728**

• **DATABASE WPI Week 199533 Derwent Publications Ltd., London, GB; AN 1995-252428 XP002278202 & JP 07 157672 A (MATSUMOTO YUSHI SEIYAKU KK) 20 juin 1995 (1995-06-20)**

**Description**

La présente invention concerne des micro-objets adhésifs non-sphériques, leur utilisation pour le maquillage et/ou le coiffage des fibres kératiniques ainsi que des compositions capillaires contenant de tels micro-objets.

Il existe de nombreux produits de coiffage sous forme de laques, de sprays, de gels ou de lotions. Ces produits sont des solutions ou dispersions de polymères qui, après évaporation de la phase solvant, assurent la fixation et le maintien de la coiffure grâce à la formation de films polymériques entourant chaque cheveu à la manière d'une gaine et grâce à l'établissement de liens physiques entre les cheveux.

Ce type de fixation de la coiffure est définitif puisque toute modification importante de la forme de la coiffure entraîne la rupture irréversible des jonctions et altère par conséquent le maintien de la coiffure. Les produits de coiffage usuels ne permettent donc pas un remodelage de la coiffure sans apport de produit coiffant supplémentaire.

Une approche pour mettre au point des compositions permettant le recoiffage ou remodelage des cheveux sans ajout de matière a consisté à associer aux polymères fixants usuels, au moins un polymère thermofusible à bas point de fusion permettant la plastification du dépôt polymérique par chauffage, remodelage et fixation de la nouvelle forme par simple refroidissement (voir demande FR 2 811 886).

Une autre approche a consisté à déposer sur les cheveux des polymères fixants plus ou moins adhésifs et/ou autoadhésifs, sous forme de films (WO98/38969) ou de particules (US 2002/0041858). Les liens adhésifs établis ainsi entre les fibres kératiniques permettent, selon le pouvoir collant des dépôts formés, une fixation forte ou souple de la chevelure, mais surtout une fixation réversible, c'est-à-dire le maintien d'une forme qu'il est possible de modifier à tout moment par un simple passage de la main ou d'un outil approprié.

La demanderesse, dans le cadre de ses recherches concernant le dépôt de particules adhésives en vue d'une fixation réversible permettant le remodelage des cheveux, a découvert qu'il était possible d'obtenir, en plus de l'effet de coiffage décrit ci-dessus, des effets optiques décoratifs très intéressants en choisissant, parmi l'ensemble des particules adhésives existantes ou envisageables, uniquement les particules non-sphériques, c'est-à-dire des particules ayant une forme telle que l'aspect de la particule varie selon la direction considérée.

Ces particules seront appelées par la suite « micro-objets non-sphériques ».

L'invention a par conséquent pour objet des micro-objets non sphériques adhésifs ayant une taille comprise entre 1 et 1000 μm, comprenant au moins une première phase solide, non-coalescente, recouverte, sur au moins une partie de sa surface, d'au moins un polymère organique adhésif. Lesdits microbjets ayant la forme d'un tétraèdre, d'un cube, d'un parallélépipède, d'une pyramide, d'un prisme, d'une plaquette, d'un cylindre, d'un cône, d'un cône tronqué ou d'une lentille concave ou convexe.

Lorsque le recouvrement est partiel, ladite première phase solide est non-coalescente.

Dans une seconde variante, les micro-objets sont constitués uniquement d'un ou de plusieurs polymères adhésifs.

L'invention a également pour objet l'utilisation de tels micro-objets non-sphériques et adhésifs pour le coiffage et/ou le maquillage des fibres kératiniques, et en particulier des cheveux.

Elle a en outre pour objet, des compositions cosmétiques pour le coiffage et/ou le maquillage des fibres kératiniques, en particulier des cheveux, comprenant de tels micro-objets non-sphériques adhésifs en dispersion dans un milieu liquide cosmétiquement acceptable.

Les micro-objets de la présente invention adhèrent à la surface des cheveux et permettent ainsi de faire coller les fibres kératiniques les unes aux autres. Comme pour les particules adhésives connues, décrites par exemple dans US 2002/0041858, cette adhérence est réversible et permet de ce fait le recoiffage des cheveux.

Les micro-objets de la présente invention apportent toutefois un effet supplémentaire lié à leur forme non-sphérique, l'adjectif « non-sphérique » englobant ici toutes les formes que l'on pourrait qualifier d'« anisotropiques », c'est-à-dire toutes les formes présentant à l'oeil, contrairement à la forme sphérique, un aspect différent selon la direction considérée. Cette variation de l'aspect en fonction de la position relative de l'oeil par rapport au micro-objet, permet l'obtention d'effets décoratifs variés et très originaux. Selon la forme, la taille, l'absorbance ou la réflectance des micro-objects utilisés, on observe en particulier des effets de brillance, de nacrage, de moirage.

La demanderesse a constaté que les effets optiques obtenus suite à l'immobilisation des micro-objets de la présente invention à la surface des cheveux étaient particulièrement intéressants lorsque les micro-objets non-sphériques avaient une forme présentant une ou plusieurs faces planes et/ou une ou plusieurs faces courbes. On peut citer à titre d'exemples de tels micro-objets, les tétraèdres, cubes, parallélépipèdes, pyramides, prismes, plaquettes, cylindres, cônes, cônes tronqués, lentilles concaves ou convexes.

Dans un mode de réalisation préféré, les micro-objets ont au moins une surface plane, et idéalement au moins deux surfaces planes.

Ces micro-objets ont en outre de préférence une forme présentant au moins un plan, axe ou centre de symétrie.

Les micro-objets comportent au moins une première phase solide non-coalescente. On entend par phase non-coalescente ou particule non-coalescente dans la présente invention des particules qui, après dépôt sur la fibre kératinique et évaporation de la phase liquide de dispersion, restent sous une forme bien individualisée sans former avec les particules adjacentes un film continu ou discontinu. Dans l'état de la technique, de tels matériaux ou particules sont également appelés non-filmogènes ou non-filmifiables.

Le fait que la phase solide formant le support pour le polymère adhésif ne coalesce pas après dépôt sur les cheveux est essentiel pour la présente invention. En effet, les micro-objets doivent conserver autant que possible leur forme particulière responsable de l'effet optique recherché.

Dans cette perspective, il convient de veiller à ce que le dépôt de polymère adhésif ne « cache » pas la forme particulière de la phase solide, c'est-à-dire que la quantité de polymère adhésif ne soit pas trop importante par rapport au volume de la phase solide non-coalescente.

La demanderesse a constaté qu'un rapport volumique polymère adhésif/phase solide non-coalescente compris entre 0,02 et 1,0, de préférence entre 0,05 et 0,8, permettait de garantir une adhérence suffisante des particules aux fibres kératiniques sans pour autant masquer la forme géométrique particulière de la phase solide.

Il n'est généralement pas indispensable pour la présente invention que la totalité de la surface des micro-objects soit couverte par le dépôt de polymère adhésif. La proportion de surface couverte par un dépôt polymérique adhésif est toutefois de préférence suffisante pour permettre non seulement l'adhérence des micro-objets de la présente invention à la fibre kératinique traitée, mais également l'adhésion de ce même micro-objet immobilisé à une deuxième fibre kératinique ou éventuellement à un deuxième micro-objet immobilisé sur une deuxième fibre.

Dans un mode de réalisation préféré des micro-objets de l'invention, la totalité ou quasi-totalité de la surface de la partie solide est couverte par le polymère adhésif.

On peut toutefois également envisager que la quantité de polymère adhésif déposé ou fixé à la surface de la phase solide non-coalescente des micro-objets de la présente invention soit juste suffisante pour faire adhérer lesdits micro-objets à la surface de la fibre sans pour autant permettre l'établissement de liens adhésifs entre différentes fibres kératiniques. Ce sera le cas par exemple lorsqu'une seule face plane des micro-objets est couverte par un dépôt de polymère adhésif.

L'effet de coiffage de tels micro-objets sera alors très faible, voire nul, et le traitement des fibres kératiniques avec de tels micro-objets servira alors principalement au maquillage des fibres.

De tels micro-objets peu adhésifs et leur utilisation pour le maquillage des fibres kératiniques sont également englobés par la présente invention.

La phase solide non-coalescente des micro-objets de la présente invention peut être de nature organique ou minérale. On peut citer à titre d'exemples de matériaux minéraux utilisables pour la phase solide non-coalescente, les métaux et alliages métalliques, les oxydes, carbures ou nitrures métalliques, les matériaux céramiques et les verres minéraux.

Lorsque la phase solide non-coalescente est une phase organique, il s'agit généralement d'un polymère organique. Pour être non-coalescent, ce polymère doit être à l'état vitreux, c'est-à-dire avoir une température de transition vitreuse significativement plus élevée que la température ambiante ou la température d'utilisation (par exemple la température du corps humain), et/ou doit être réticulé.

La température de transition vitreuse des polymères organiques utilisables pour la phase solide est de préférence supérieure à 40 °C, de préférence supérieure à 60 °C et en particulier comprise entre 80 °C et 200 °C.

Parmi ces polymères, on peut citer de façon non-exhaustive, le polystyrène, le poly(acétate de vinyle), le poly($\alpha$-méthylstyrène), le poly(acrylamide), le poly(acrylonitrile), le poly(chlorure de vinyle), les copolymères à base de styrène et de (méth)acrylate d'alkyle en $C_{1-4}$, les copolymères à base de styrène et d'acrylamide, les copolymères à base de styrène et d'acrylonitrile, les copolymères à base de styrène et d'acétate de vinyle, les copolymères à base d'acrylamide et de (méth)acrylates d'alkyle en $C_{1-4}$, les copolymères à base d'acrylonitrile et de (méth)acrylate d'alkyle en $C_{1-4}$, les copolymères à base d'acrylonitrile et d'acrylamide, les terpolymères à base de styrène, d'acrylonitrile et d'acrylamide, le poly(méthacrylate de méthyle), le poly(méthacrylate d'éthyle), les copolymères styrène/butadiène, styrène/acide acrylique, styrène/vinylpyrrolidone et butadiène/acrylonitrile.

La phase solide non-coalescente des micro-objets de la présente invention porte, sur une partie ou sur la totalité de sa surface, un dépôt de polymère organique adhésif.

Le polymère adhésif peut être immobilisé à la surface de la phase solide de préférence non-coalescente par des liaisons chimiques covalentes (greffage) ou par des interactions physico-chimiques faibles telles que l'interaction hydrophobe, les liaisons hydrogène et les forces de van der Waals (adsorption).

Dans une seconde variante de l'invention, les micro-objets sont constitués intégralement d'un ou de plusieurs polymères organiques adhésifs.

Le caractère adhésif d'un polymère organique est généralement lié à température de transition vitreuse de celui-ci. Une condition nécessaire mais non suffisante pour qu'un polymère soit adhésif est une température de transition vitreuse ($T_g$) significativement inférieure à la température ambiante ou à la température d'utilisation. Les polymères organiques adhésifs utilisés pour la préparation des micro-objets de la présente invention ont de préférence une température de transition vitreuse inférieure ou égale à 10 °C, de préférence inférieure ou égale à 0 °C.

Les polymères organiques adhésifs utilisés dans la présente invention, ont de préférence une auto-adhésivité telle que la force de traction ($F_{max}$ (en N)) nécessaire pour séparer deux surfaces enduites dudit polymère, est supérieure à 1 N, de préférence supérieure à 3 N et en particulier supérieure à 5 N. Cette force de traction $F_{max}$ est mesurée dans les conditions suivantes : Deux disques ayant chacun une surface de 38 mm$^2$, en un matériau solide, rigide, inerte et non-absorbant, de préférence du verre, sont enduits d'une couche du polymère adhésif à tester. Le polymère est déposé en une quantité de 519 μg/mm$^2$ à partir d'une solution dans un solvant approprié. Après évaporation dudit solvant pendant 24 heures à 22 °C sous une humidité relative de 50 %, les deux surfaces enduites des disques sont superposées et les disques pressés l'un contre l'autre pendant 20 secondes à une pression de 3 Newtons à l'aide d'un extensomètre LLOYD modèle LR5K.

On tire alors sur les disques collés de manière à les éloigner l'un de l'autre à une vitesse de 20mm/minute et l'on enregistre en continu la force de traction. La force de traction maximale, enregistrée au moment de la séparation des deux surfaces, appelée $F_{max}$ caractérise l'autoadhésivité du polymère. Plus cette force est importante, plus l'auto-adhésivité du polymère est grande.

Les polymères adhésifs utilisables pour la présente invention peuvent également être caractérisés par leur adhésivité sur un matériau inerte, tel que le verre. Cette adhésivité peut être exprimée sous forme d'énergie ($E_s$) fournie par le même extensomètre (LLOYD modèle LR5K) pour séparer une surface de 38 mm$^2$ enduite, dans les conditions ci-dessus (519 mg/mm$^2$, séchage 24 heures à 22 °C, 50 % RH) d'un polymère adhésif, d'une surface en verre poli, après compression de ces deux surfaces pendant 30 secondes avec une force de 3 Newton. Comme précédemment, la vitesse de traction est de 20 mm/min.

Cette énergie $E_s$, correspondant à la somme de travail fourni jusqu'au décollement, peut être calculée selon la formule suivante :

$$\int_{Xs1 + 0,05}^{Xs2} F(x)dx$$

où
F(x) est la force nécessaire pour produire un déplacement (x),
Xs1 est le déplacement (exprimé en mm) produit par la force de traction maximale, et
Xs2 est le déplacement (exprimé en mm) produit par la force de traction permettant la séparation totale des deux surfaces.

Pour les polymères adhésifs utilisés dans la présente invention, l'énergie de séparation $E_s$ est de préférence au plus égale 300 μJ, de préférence au plus égale à 250 μJ.

La nature chimique des polymères organiques adhésifs n'est pas déterminante pour la présente invention pour autant que le dépôt de polymère présente les caractéristiques d'adhésivité et/ou d'auto-adhésivité ci-dessus. Ces polymères adhésifs pourront être réticulés ou non. Pour trouver des exemples concrets de polymères adhésifs, on pourra se référer aux demandes suivantes décrivant des particules ou des polymères adhésifs :

WO98/38969, FR 2833960 (Polyuréthannes cationiques ou amphotères auto-adhésifs), FR 2833959 (Polymères radicalaires cationiques ou amphotères autoadhésifs).

Les micro-objets de la présente invention ont une taille comprise entre 1 μm et 1000 μm, de préférence comprise entre 10 et 900 μm. Cette taille est celle de la dimension la plus grande de chaque micro-objet. Il est bien entendu que, lorsqu'il s'agit d'une population de micro-objets de différentes tailles, la taille indiquée correspond à la taille moyenne de la population.

La présente invention a également pour objet des compositions de coiffage et/ou de maquillage des fibres kératiniques. Dans ces compositions capillaires, les micro-objets sont dispersés dans un milieu liquide cosmétiquement acceptable. Le milieu liquide cosmétiquement acceptable est formé généralement d'eau et/ou d'un ou plusieurs solvants organiques utilisés couramment en cosmétique. Ces solvants cosmétiquement acceptables sont des liquides à température ambiante et à pression atmosphérique. Ces solvants sont de préférence des monoalcools, des polyols ou des éthers de ces alcools ou polyols. On peut citer par exemple l'éthanol, l'isopropanol, le glycérol, le propylèneglycol, l'éther monométhylique de propylèneglycol.

Ce milieu liquide devra, bien entendu, être choisi de manière à ne pas dissoudre le polymère organique adhésif et la phase solide de préférence non-coalescente.

La concentration des micro-objets dans les compositions capillaires de la présente invention est de préférence comprise entre 1 et 90 % en poids, en particulier entre 1 et 80 % en poids, et idéalement entre 1 et 50 % en poids, rapporté au poids total de la composition.

Les compositions capillaires de la présente invention peuvent contenir en outre des principes actifs et adjuvants cosmétiques utilisés couramment dans le domaine capillaire. Ces additifs sont choisis par exemple parmi les vitamines, les acides aminés, les oligopeptides, les peptides, les protéines hydrolysées ou non, modifiées ou non, les enzymes, les acides et alcools gras ramifiés ou non, les cires animales, végétales ou minérales, les céramides et les pseudo-céramides, les acides organiques hydroxylés, les filtres UV, les agents anti-oxydants et les agents anti-radicaux libres, les agents chélatants, les agents antipelliculaires, les agents régulateurs de séborrhée, les agents apaisants, les agents tensioactifs cationiques, anioniques, non-ioniques ou amphotères, les polymères cationiques, anioniques, neutres ou amphotères, les silicones organomodifiées ou non, les huiles minérales, végétales ou animales, les polyisobutènes et poly($\alpha$-oléfines), les esters gras, les agents colorants tels que les colorants directs et les pigments.

Les micro-objets de la présente invention peuvent être préparés selon n'importe quel procédé approprié permettant la fabrication de tels objets de petite taille ayant une forme particulière.

Dans la première variante de l'invention, la phase solide non-coalescente peut être préparée par exemple par coulée d'un film mince à partir d'une solution d'un polymère, séchage et découpage de ce film par un outil approprié tel qu'un rayon laser, une lame ou un emporte-pièce. Le film polymérique peut éventuellement être soumis à une étape de chauffage ou d'irradiation en vue de sa réticulation.

Le dépôt de polymère adhésif peut être appliqué sur le film avant découpage par exemple par pulvérisation, immersion, centrifugation ou étalement au moyen d'un rouleau ou d'une racle. On peut également envisager le dépôt de la couche de polymère adhésif sur les micro-objets obtenus après découpage du film. Cette application peut se faire par exemple par pulvérisation ou immersion.

La phase solide non-coalescente peut encore être préparée par extrusion de feuilles ou de brins très fins à section circulaire ou polygonale, à partir d'un polymère à l'état fondu ou d'une solution de polymère ayant une viscosité appropriée. Ces brins ou feuilles seront ensuite découpés pour donner des micro-objets selon l'invention. La couche de polymère adhésif peut être appliquée sur la phase solide non-coalescente de la manière décrite ci-dessus ou encore par co-extrusion du polymère adhésif et du polymère formant la phase solide non-coalescente.

Dans la seconde variante de l'invention, on peut polymériser le polymère adhésif sur place et procéder à son découpage selon les formes voulues par les mêmes techniques que décrites précédemment.

## Exemple 1

Composition selon l'invention

| | |
|---|---|
| Particules plaquettaires en polystyrène totalement recouvertes de polymère AQ® 1350* (20 $\mu$m x 5 $\mu$m) | 2g |
| Acide polyacrylique réticulé | 0,3g |
| Eau | 100g |
| * polyester ramifié sulfoné hydrodispersible commercialisé par la société Eastman Chemical Company | |

Le polystyrène peut être remplacé par du nitrure de bore.

## Revendications

1. Micro-objet non-sphérique adhésif, ayant une taille comprise entre 1 et 1000 $\mu$m, comprenant au moins une première phase solide, non-coalescente recouverte, sur au moins une partie de sa surface, d'au moins un polymère organique adhésif ledit microobjet ayant la forme d'un tétraèdre, d'un cube, d'un parallélépipède, d'une pyramide, d'un prisme, d'une plaquette, d'un cylindre, d'un cône, d'un cône tronqué ou d'une lentille concave ou convexe.

2. Micro-objet non-sphérique selon la revendication 1 **caractérisé par le fait que** le polymère organique adhésif a une température de transition vitreuse ($T_g$) inférieure à 10 °C, de préférence inférieure à 0°C.

3. Micro-objet non-sphérique selon la revendication 1 ou 2 **caractérisé par le fait que** le polymère organique adhésif a une auto-adhésivité telle que la force de traction ($F_{max}$ (en N) nécessaire pour séparer deux surfaces enduites dudit polymère, est supérieure à 1 N, de préférence supérieure à 3 N et en particulier supérieure à 5 N.

4. Micro-objet non-sphérique selon l'une quelconque des revendications précédentes, **caractérisé par le fait que** le polymère organique adhésif a une adhésivité telle que l'énergie $E_s$ fournie pour séparer une surface de 38 mm$^2$ enduite dudit polymère d'une surface en verre poli est inférieure à 300 $\mu$J, de préférence inférieure à 250 $\mu$J.

5. Micro-objet non-sphérique selon la revendication ou **caractérisé par le fait qu'**il présente au moins un centre, axe ou plan de symétrie.

**6.** Micro-objet non-sphérique selon l'une des revendications 1, à 5 **caractérisé par le fait que** la première phase solide est formée par un matériau minéral choisi parmi les métaux et alliages métalliques, les oxydes, carbures ou nitrures métalliques, les matériaux céramiques et les verres minéraux.

**7.** Microobjet non-sphérique selon l'une quelconque des revendications 1 *à* 5 **caractérisé par le fait que** la première phase solide est formée par un polymère organique ayant une température de transition vitreuse supérieure à 40 °C, de préférence supérieure à 60 °C , et en particulier comprise entre 80. °C et 200 °C.

**8.** Micro-objet selon l'une quelconque des revendications 1 à 6 **caractérisé par le fait que** la première phase solide est formée par un polymère organique réticulé.

**9.** Micro-objet selon l'une quelconque des revendications précédentes **caractérisé par le fait que** le polymère adhésif recouvre la totalité ou la quasi-totalité de la surface dudit micro-objet.

**10.** Micro-objet selon l'une quelconque des revendications précédentes **caractérisé par le fait que** le polymère adhésif est fixé sur la phase solide par des liaisons chimiques covalentes et/ou par des interactions physico-chimiques faibles.

**11.** Micro-objet selon l'une quelconque des revendications précédentes, **caractérisé par le fait que** le rapport volumique du polymère adhésif à la phase solide est compris entre 0,02 et 1,0, de préférence entre 0,05 et 0,8.

**12.** Utilisation des micro-objets non sphérique, ayant une taille comprise entre 1 et 1000 $\mu$m et ayant la forme d'un tétraèdre, d'un cube, d'un parallélépipède, d'une pyramide, d'un prisme, d'une plaquette, d'un cylindre, d'un cône, d'un cône tronqué ou d'une lentille concave ou convexe, comprenant au un polymère adhésif, pour le maquillage des fibres kératiniques, en particulier des cheveux.

**13.** utilisation des micro-objets selon l'une quelconque des revendications 1 à 11, pour le maquillage et/ou le coiffage des fibres kératiniques, et particulier des cheveux. 26-04-2006 kératiniques, en particulier des cheveux

**14.** Composition cosmétique pour le coiffage et/ou maquillage des fibres kératiniques, comprenant des micro-objets selon l'une quelconque des revendications 1 à 11, en dispersion dans un milieu liquide cosmétiquement acceptable.

**15.** Composition selon la revendication 14 **caractérisée par le fait que** la concentration des micro-objets est comprise entre 1 et 90 % en poids, de préférence entre 1 et 80 % en poids, et en particulier entre 1 et 50 % en poids rapporté au poids total de la composition cosmétique.

**Claims**

**1.** Adhesive nonspherical microobject having a size of between 1 and 1000 $\mu$m and comprising at least one first noncoalescent solid phase covered, over at least a part of its surface, with at least one adhesive organic polymer, the said microobject having the shape of a tetrahedron, of a cube, of a parallelepiped, of a pyramid, of a prism, of a sheet, of a cylinder, of a cone, of a truncated cone or of a concave or convex lens.

**2.** Nonspherical microobject according to Claim 1, **characterized in that** the adhesive organic polymer has a glass transition temperature (Tg) of less than 10°C, preferably of less than 0°C.

**3.** Nonspherical microobject according to Claim 1 or 2, **characterized in that** the adhesive organic polymer has a self-adhesiveness such that the tensile force ($F_{max}$ (in N) ) necessary to separate two surfaces coated with the said polymer is greater than 1N, preferably greater than 3N and in particular greater than 5N.

**4.** Nonspherical microobject according to any one of the preceding claims, **characterized in that** the adhesive organic polymer has an adhesiveness such that the energy $E_s$ supplied to separate a surface with an area of 38 mm$^2$ coated with the said polymer from a surface made of polished glass is less than 300 $\mu$J, preferably less than 250 $\mu$J.

**5.** Nonspherical microobject according to Claim 1, **characterized in that** it exhibits at least one centre, axis or plane of symmetry.

**6.** Nonspherical microobject according to one of Claims 1 to 5, **characterized in that** the first solid phase is formed by an inorganic material chosen from metals and metal alloys, metal oxides, carbides or nitrides, ceramic materials and mineral glasses.

7. Nonspherical microobject according to any one of Claims 1 to 5, **characterized in that** the first solid phase is formed by an organic polymer having a glass transition temperature of greater than 40°C, preferably of greater than 60°C and in particular of between 80°C and 200°C.

8. Microobject according to any one of Claims 1 to 6, **characterized in that** the first solid phase is formed by a crosslinked organic polymer.

9. Microobject according to any one of the preceding claims, **characterized in that** the adhesive polymer covers all or virtually all of the surface of the said microobject.

10. Microobject according to any one of the preceding claims, **characterized in that** the adhesive polymer is fixed to the solid phase by covalent chemical bonds and/or by weak physicochemical interactions.

11. Microobject according to any one of the preceding claims, **characterized in that** the ratio by volume of the adhesive polymer to the solid phase is between 0.02 and 1.0, preferably between 0.05 and 0.8.

12. Use of the nonspherical microobjects having a size of between 1 and 1000 $\mu$m and having the shape of a tetrahedron, of a cube, of a parallelepiped, of a pyramid, of a prism, of a sheet, of a cylinder, of a cone, of a truncated cone or of a concave or convex lens, comprising at least one adhesive polymer, for making up keratinous fibres, in particular the hair.

13. Use of the micro-objects according to any one of Claims 1 to 11 for making up and/or styling keratinous fibres, in particular the hair.

14. Cosmetic composition for styling and/or making up keratinous fibres, comprising micro-objects according to any one of Claims 1 to 11 in dispersion in a cosmetically acceptable liquid medium.

15. Composition according to Claim 14, **characterized in that** the concentration of the micro-objects is between 1 and 90% by weight, preferably between 1 and 80% by weight and in particular between 1 and 50% by weight, with respect to the total weight of the cosmetic composition.

**Patentansprüche**

1. Nicht-sphärisches, adhäsives Mikroobjekt, das eine Grösse zwischen 1 und 1000 $\mu$m aufweist, umfassend mindestens eine erste nicht-koaleszente Festphase, die, auf mindestens einem Anteil ihrer Fläche von einem organischen, adhäsiven Polymer bedeckt ist, wobei das Mikroobjekt die Form eines Tetraeders, eines Würfels, eines Quaders, einer Pyramide, eines Prismas, einer Platte, eines Zylinders, eines Kegels, eines Kegelstumpfs oder einer konkaven oder konvexen Linse aufweist.

2. Nicht-sphärisches Mikroobjekt nach Anspruch 1, **dadurch gekennzeichnet, dass** das organische adhäsive Polymer eine Glasübergangstemperatur (Tg) aufweist, die kleiner als 10 °C ist, vorzugsweise kleiner als 0 °C.

3. Nicht-sphärisches Mikroobjekt nach Anspruch 1 oder 2, **dadurch gekennzeichnet, dass** das organische adhäsive Polymer eine solche Autoadhäsivität aufweist, dass die zur Trennung zweier mit diesem Polymer beschichteter Flächen benötigte Zugkraft ($F_{max}$ (in N)) grösser als 1 N, vorzugsweise grösser als 3 N, insbesondere grösser als 5 N ist.

4. Nicht-sphärisches Mikroobjekt nach einem der vorherigen Ansprüche, **dadurch gekennzeichnet, dass** das organische adhäsive Polymer eine solche Adhäsivität aufweist, dass die zur Trennung einer mit diesem Polymer beschichteten Fläche von 38 mm$^2$ von einer Flachglasfläche aufgebrachte Energie $E_s$ kleiner als 300 $\mu$J, vorzugsweise kleiner als 250 $\mu$J ist.

5. Nicht-sphärisches Mikroobjekt nach Anspruch 1, **dadurch gekennzeichnet, dass** es mindestens ein Symmetriezentrum, eine Symmetrieachse oder eine Symmetrieebene aufweist.

6. Nicht-sphärisches Mikroobjekt nach einem der Ansprüche 1 bis 5, **dadurch gekennzeichnet, dass** die erste Festphase von einem mineralischen Material gebildet wird, das ausgewählt ist aus Metallen und Metalllegierungen, Metalloxiden, -karbiden oder -nitriden, keramischen Materialien und Mineralgläsern.

7. Nicht-sphärisches Mikroobjekt nach einem der Ansprüche 1 bis 5, **dadurch gekennzeichnet, dass** die erste Festphase von einem organischen Polymer gebildet wird, welches eine Glasübergangstemperatur aufweist, die größer als 40 °C, vorzugsweise größer als 60 °C ist und insbesondere zwischen 80 °C und 200 °C liegt.

8. Mikroobjekt nach einem der Ansprüche 1 bis 6, **dadurch gekennzeichnet, dass** die erste Festphase von einem vernetzten organischen Polymer gebildet wird.

9. Mikroobjekt nach einem der vorherigen Ansprüche, **dadurch gekennzeichnet, dass** das organische Polymer die Gesamtheit oder beinahe die Gesamtheit der Fläche des Mikroobjekts bedeckt.

10. Mikroobjekt nach einem der vorherigen Ansprüche, **dadurch gekennzeichnet, dass** das adhäsive Polymer auf der Festphase durch kovalente chemische Bindungen und/oder schwache physikalisch-chemische Wechselwirkungen befestigt ist.

11. Mikroobjekt nach einem der vorherigen Ansprüche, **dadurch gekennzeichnet, dass** das Volumenverhältnis des adhäsiven Polymers zu der Festphase zwischen 0,02 und 1,0, vorzugsweise zwischen 0,05 und 0,8 liegt.

12. Verwendung von nicht-sphärischen Mikroobjekten, die eine Größe zwischen 1 und 1000 μm aufweisen und die Form eines Tetraeders, eines Würfels, eines Quaders, einer Pyramide, eines Prismas, einer Platte, eines Zylinders, eines Kegels, eines Kegelstumpfs oder einer konkaven oder konvexen Linse aufweisen, umfassend mindestens ein adhäsives Polymer zum Schminken von Keratinfasern, insbesondere von Haaren.

13. Verwendung von Mikroobjekten nach einem der Ansprüche 1 bis 11 zum Schminken und/oder zum Formen von Keratinfasern, insbesondere von Haaren.

14. Kosmetische Zusammensetzung zum Formen und/oder Schminken von Keratinfasern, welche Mikroobjekte nach einem der Ansprüche 1 bis 11 dispergiert in einem kosmetisch verträglichen flüssigen Medium aufweist.

15. Zusammensetzung nach Anspruch 14, **dadurch gekennzeichnet, dass** die Konzentration der Mikroobjekte zwischen 1 und 90 Gew.%, vorzugsweise zwischen 1 und 80 Gew.% und insbesondere zwischen 1 und 50 Gew.% bezüglich des Gesamtgewichts der kosmetischen Zusammensetzung beträgt.